# EUROPEAN PATENT SPECIFICATION

(11) **EP 3 383 173 B1**
(45) Date of publication and mention of the grant of the patent: **25.11.2020**
(21) Application number: 16802036.0
(22) Date of filing: 28.11.2016
(51) Int. Cl.: A01K 67/027, C12N 5/0786, A61K 39/395, C07K 16/28

(54) **GENERATION OF HUMAN MACROPHAGES IN IMMUNODEFICIENT MICE**
ERZEUGUNG VON MENSCHLICHEN MAKROPHAGEN IN IMMUNDEFIZIENTE MÄUSE
PRODUCTION DE MACROPHAGES HUMAINS DANS DES SOURIS IMMUNODÉFICIENTES

(30) Priority: 30.11.2015 EP 15196914
(43) Date of publication of application: 10.10.2018
(73) Proprietor: F. Hoffmann-La Roche AG, 4070 Basel (CH)
(72) Inventor: ECKMANN, Jan, 81545 Muenchen (DE); HOVES, Sabine, 82392 Habach (DE); RIES, Carola, 82377 Penzberg (DE)
(74) Representative: Burger, Alexander
(86) International application number: PCT/EP2016/078935
(87) International publication number: WO 2017/093155

(56) References cited:
- WO-A1-96/04785
- ANTHONY RONGVAUX ET AL: "Development and function of human innate immune cells in a humanized mouse model", NATURE BIOTECHNOLOGY, 16 March 2014 (2014-03-16), pages 1-9, XP055112690, ISSN: 1087-0156, DOI: 10.1038/nbt.2858 cited in the application
- JONATHAN ELEGHEERT ET AL: "Extracellular Complexes of the Hematopoietic Human and Mouse CSF-1 Receptor Are Driven by Common Assembly Principles", STRUCTURE, CURRENT BIOLOGY LTD., PHILADELPHIA, PA, US, vol. 19, no. 12, 6 October 2011 (2011-10-06), pages 1762-1772, XP028338759, ISSN: 0969-2126, DOI: 10.1016/J.STR.2011.10.012 [retrieved on 2011-10-29] cited in the application
- M. A. BREHM ET AL: "Overcoming Current Limitations in Humanized Mouse Research", JOURNAL OF INFECTIOUS DISEASES. JID, vol. 208, no. suppl 2, 22 October 2013 (2013-10-22), pages S125-S130, XP055271223, CHICAGO, IL. ISSN: 0022-1899, DOI: 10.1093/infdis/jit319
- LEONARD D. SHULTZ ET AL: "Humanized mice for immune system investigation: progress, promise and challenges", NATURE REVIEWS IMMUNOLOGY, vol. 12, no. 11, 1 November 2012 (2012-11-01), pages 786-798, XP055064740, ISSN: 1474-1733, DOI: 10.1038/nri3311
- ANTHONY RONGVAUX ET AL: "Human Hemato-Lymphoid System Mice: Current Use and Future Potential for Medicine", ANNUAL REVIEW OF IMMUNOLOGY., vol. 31, no. 1, 21 March 2013 (2013-03-21) , pages 635-674, XP055271225, US ISSN: 0732-0582, DOI: 10.1146/annurev-immunol-032712-095921
- S. TANAKA ET AL: "Development of Mature and Functional Human Myeloid Subsets in Hematopoietic Stem Cell-Engrafted NOD/SCID/IL2r KO Mice", THE JOURNAL OF IMMUNOLOGY, vol. 188, no. 12, 15 June 2012 (2012-06-15), pages 6145-6155, XP055052563, ISSN: 0022-1767, DOI: 10.4049/jimmunol.1103660

## Description

The invention relates to the use of non-anti-human antibodies to stimulate the generation of human macrophages in immunodeficient non-human hosts, reconstituted with human immune system. According methods for the generation and host models with human xenografts as well as their use for cancer immunotherapy evaluation are also included.

### Background of the Invention

Poor reconstitution of the human innate immune system, in particular myeloid and NK cells, represent one of the major limitations in using HIS (human immune system) mice for drug development in cancer. As reported before (Li Y, et al, Journal of immunology, 191 (2013) 3192-3199; Rathinam C, et al, Blood 118 (2011) 3119-3128; Rongvaux A, et al, Nature biotechnology 32 (2014) 364-372), human white blood cells in are biased towards the lymphoid lineage (B and T cells) in HIS mice whereas human CD11b⁺ myeloid cells including CD14⁺CD33⁺ monocytes are highly underrepresented.

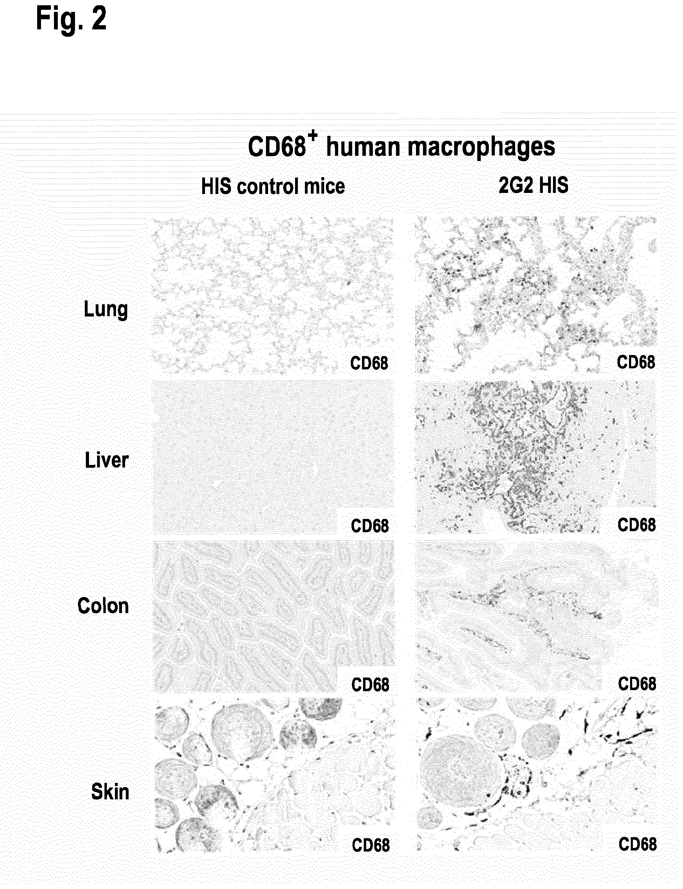

In consequence, differentiated human tissue macrophages are present only at extreme low frequencies also in immunodeficient mice which are reconstituted with human immune system (HIS) (Fig.2 left row).

The underrepresentation of monocytes and macrophages can be overcome by expressing the human macrophage colony stimulating factor (huCSF-1) which indicates the essential role of CSF-1R signaling for differentiation and survival of human monocytes and macrophages in these mice (Li Y, et al, Journal of immunology, 191 (2013) 3192-3199; Rathinam C, et al, Blood 118 (2011) 3119-3128; Rongvaux A, et al, Nature biotechnology 32 (2014) 364-372). Murine CSF-1 (muCSF-1) binds to the huCSF-1R with a 500-fold lower affinity compared to huCSF-1 (Elegheert J, et al, Structure 19 (2011) 1762-1772); hence, muCSF-1 was claimed to not cross-react with huCSF-1R.

### Summary of the Invention

The invention is defined in the appended claims and any subject matter not falling within the scope of the claims is for information only.

The inventors demonstrate for the first time that antibody-mediated blockade of muCSF-1R is associated with a significant reduction of host macrophages and 1000-fold increase of muCSF-1 in humanized mice.

Here the inventors found out that treatment of immunodeficient mice, reconstituted with human immune system (HIS mice) with an *anti-mouse CSF1R* antibody (which is not cross-reactive with human CSF-1R) results in 1) depletion of mouse macrophages and 2) surprisingly also in improved reconstitution of *human macrophages* in these mice.

These changes can be harnessed to increase the frequencies of human monocytes in peripheral blood (∼25% of total human CD45⁺ cells) as well as *de novo* infiltration of human macrophages in lymphoid and non-lymphoid tissues. In turn, enhanced reconstitution can be reversed *in vivo* by blocking muCSF-1 resulting in significantly reduced human tissue macrophages.

Notably, human macrophages can be induced by anti-CSF-lR antibody treatment at different time points after reconstitution independently of the initial overall human reconstitution level.

Moreover, in HIS mice xenografted with human tumor cells, *anti-mouse CSF1R* antibody treatment results in replacement of mouse by human macrophages tumor associated macrophages.

Stimulation of human macrophage generation in tumor bearing HIS mice (OVCAR5 and HT29), resulted in slower tumor growth in the presence of human macrophages. In depth analysis revealed, that tumors are infiltrated by both, M1- and M2-like, macrophage subtypes, and accompanied by enhanced T cell infiltrate. In summary, we developed a novel humanized model which offers now the opportunity to evaluate cancer immunotherapies in the context of a pre-existing innate and adaptive immune cell infiltrate.

So the present invention relates to the use an antibody which specifically binds to mouse colony-stimulating factor 1 receptor (CSF-1R) and which does not crossreact with human CSF-1R to stimulate the generation of human macrophages in immunodeficient mouse, wherein immunodeficient mouse is reconstituted with human CD34 positive hematopoietic stem cells and wherein the antibody binds to human CSF-1R with a binding affinity of KD-value of 1.0 x 10⁻⁶ mol/l or higher at 25°C.

In one embodiment the mouse macrophages in the mouse are reduced by at least 40% (in one embodiment by at least 50% in peripheral blood and bone marrow, in one embodiment by at least 60% in peripheral blood and bone marrow) compared to an IgG control antibody (which does not bind to non-human host CSF-1R).

In one embodiment the human macrophages are generated in non-lymphoid tissues.

In one embodiment the human macrophages are generated in lymphoid tissues.

In one embodiment the mouse has been xenografted with human tumor cells (either subcutaneously or orthotopically) . And the anti-mouse CSF-1R antibody is used for the generation of human tumor associated macrophages (TAMs).

### Description of the Figures

- **Figure 1**: Generation of immunodeficient non-human host (e.g. a mouse), reconstituted with human immune system (e.g. with reconstituted with human CD34 positive (CD34+) hematopoietic stem cells.
- **Figure 2**: Generation of human macrophages in nolymhoid tissues of immunodeficient mice reconstituted with human immune system.
- **Figure 3**: Generation of human tumor associated macrophages (TAMs) in immunodeficient mice reconstituted with human immune system.
- **Figure 4**: Significant reduction of mouse macrophages in immunodeficient mice, reconstituted with human immune system (A) Level of mouse M-CSF in serum of HIS BRG mice treated with 2G2 or MOPC21 (n=8-9 mice).
(B+C) Frequency of Ly6C⁺⁺⁺CSF1R⁺ mouse monocytes or macrophages within mouse CD45⁺ cells in peripheral blood of humanized mice injected with 4 doses of control (MOPC21) or anti-mouse CSF1R (2G2) antibody determined by FACS analysis (n= 8-10 mice; 2way Anova; ^{∗}P < 0.05).
(D+E) Frequency of Ly6C⁺⁺⁺CSF1R⁺ mouse monocytes or macrophages within mouse CD45⁺ cells in bone marrow or spleen of humanized mice injected with 4 doses of control (MOPC21) or anti-mouse CSF1R (2G2) antibody determined by FACS analysis (n= 8-9 mice; unpaired Student's t-test).
(F+G) Immunohistochemical staining of mouse F480 on representative spleen or bone marrow sections of humanized mice injected with 4 doses of control (MOPC21) or anti-mouse CSF1R (2G2) antibody.

### Detailed Description of the Invention

The invention is defined in the appended claims and any subject matter not falling within the scope of the claims is for information only.

Many tumors are characterized by a prominent immune cell infiltrate, including macrophages. Initially, the immune cells were thought to be part of a defense mechanism against the tumor, but recent data support the notion that several immune cell populations including macrophages may, in fact, promote tumor progression. Macrophages are characterized by their plasticity. Depending on the cytokine microenvironment, macrophages can exhibit so-called M1 or M2-subtypes. M2 macrophages are engaged in the suppression of tumor immunity. They also play an important role in tissue repair functions such as angiogenesis and tissue remodeling which are coopted by the tumor to support growth. In contrast to tumor promoting M2 macrophages, M1 macrophages exhibit antitumor activity via the secretion of inflammatory cytokines and their engagement in antigen presentation and phagocytosis (Mantovani, A. et al., Curr. Opin. Immunol. 2 (2010) 231-237).

By secreting various cytokines such as colony stimulating factor 1 (CSF-1) and IL-10, tumor cells are able to recruit and shape macrophages into the M2- subtype, whereas cytokines such as granulocyte macrophage colony stimulating factor (GM-CSF), IFN-gamma program macrophages towards the M1 subtype. Using immunohistochemistry, it is possible to distinguish between a macrophage subpopulation co-expressing CD68 and CD163, which is likely to be enriched for M2 Macrophages, and a subset showing the CD68+/MHC II+, or CD68+/CD80+ immunophenotype, likely to include M1 macrophages. Cell shape, size, and spatial distribution of CD68 and CD163 positive macrophages is consistent with published hypotheses on a tumor-promoting role of M2 macrophages, for example by their preferential location in tumor intersecting stroma, and vital tumor areas. In contrast, CD68+/MHC class II+ macrophages are ubiquitously found. Their hypothetical role in phagocytosis is reflected by clusters of the CD68+/MHC class II+, but CD163-immunophenotype near apoptotic cells and necrotic tumor areas.

Consistent with a role for CSF-1 in driving the pro-tumorigenic function of M2 macrophages, high CSF-1 expression in rare sarcomas or locally aggressive connective tissue tumors, such as pigmented villonodular synovitis (PVNS) and tenosynovial giant cell tumor (TGCT) due in part to a translocation of the CSF-1 gene, leads to the accumulation of monocytes and macrophages expressing the receptor for CSF-1, the colony-stimulating factor 1 receptor (CSF-1R) forming the majority of the tumor mass (West, R.B. et al., Proc. Natl. Acad. Sci. USA 3 (2006) 690-695). These tumors were subsequently used to define a CSF-1 dependent macrophage signature by gene expression profiling. In breast cancer and leiomyosarcoma patient tumors this CSF-1 response gene signature predicts poor prognosis (Espinosa, I. et al., Am. J. Pathol. 6 (2009) 2347-2356; Beck, A. et al., Clin. Cancer Res. 3 (2009) 778-787).

The mouse CSF-1R (Swiss Prot Accesion Nos. P09581; Q3U3P1; Q9DBH9; CSF-1 receptor; synonyms: M-CSF receptor; Macrophage colony-stimulating factor 1 receptor, c-fms) has 76% and 75% homology to the v-fms and human proteins, respectively. The predicted membrane protein, c-fms, has an amino terminal signal sequence and external, transmembrane and cytoplasmic domains. The homology between murine c-fms and v-fms or human c-fms is the strongest (90%-95%) in the cytoplasmic domain and weakest (59%-63%) in the external domain (Rothwell et al, Oncogene Res. 1 (1987) 311-324; and Sherr et al, Cell 41 (1985) 665-676.)

The human CSF-1R (CSF-1 receptor; synonyms: M-CSF receptor; Macrophage colony-stimulating factor 1 receptor, Fms proto-oncogene, c-fms)) is known since 1986 (Coussens, L., et al., Nature 320 (1986) 277-280). CSF-1R is a growth factor and encoded by the c-fms proto-oncogene (reviewed e.g. in Roth, P. and Stanley, E.R., Curr. Top. Microbiol. Immunol. 181 (1992) 141-167).

CSF-1R is the receptor for the CSF-1R ligands CSF-1 (macrophage colony stimulating factor, also called M-CSF) (SEQ ID No.: 86) and IL-34 (SEQ ID No.: 87) and mediates the biological effects of these cytokines (Sherr, C.J., et al., Cell 41 (1985) 665-676; Lin, H., et al., Science 320 (2008) 807-811). The cloning of the colony stimulating factor-1 receptor (also called c-fms) was described for the first time in Roussel, M.F., et al., Nature 325 (1987) 549-552. In that publication, it was shown that CSF-1R had transforming potential dependent on changes in the C-terminal tail of the protein including the loss of the inhibitory tyrosine 969 phosphorylation which binds Cbl and thereby regulates receptor down regulation (Lee, P.S., et al., Embo J. 18 (1999) 3616-3628).

The main biological effects of CSF-1R signaling are the differentiation, proliferation, migration, and survival of hematopoietic precursor cells to the macrophage lineage (including osteoclast).

As used herein, "an antibody which specifically binds to mouse CSF-1R" refers to an antibody specifically binding to the mouse CSF-1R antigen with a binding affinity of KD-value of 1.0 x 10⁻⁸ mol/l or lower at 25°C, in one embodiment of a KD-value of 1.0 x10⁻⁹ mol/l or lower at 25°C. The binding affinity is determined with a standard binding assay at 25°C, such as surface plasmon resonance technique (BIAcore®, GE-Healthcare Uppsala, Sweden). Thus an "antibody binding to mouse CSF-1R" as used herein refers to an antibody specifically binding to the mouse CSF-1R antigen with a binding affinity of KD 1.0 x 10⁻⁸ mol/l or lower (preferably 1.0 x 10⁻⁸ mol/l - 1.0 x 10⁻¹² mol/l) at 25°C).

As used herein, the term "does not crossreact with human CSF-1R" or "does not specifically bind to human CSF-1R" refers to an antibody which exhibits not specifically binding interaction to the human CSF-1R antigen (binding affinity of KD-value of 1.0 x 10⁻⁶ mol/l or higher at 25°C in a standard binding assay, such as surface plasmon resonance technique (BIAcore®, GE-Healthcare Uppsala, Sweden). The IgG control antibody used in the examples does not (specifically) bind to non-human host CSF-1R, which means it binds to another unrelated antigen or shows no binding all.

The term non-human host refers to a non human animal. Preferably said non-human hosted or non-human animal is a rodent and more preferably a mouse, rat or rabbit. In the invention said non-human hosted or non-human animal is an immunodeficient mouse. In one preferred embodiment the mouse is a Rag2-/-yc-/-, nude Rag 2-/-, NOD (NOD means according to the invention preferably NOD.Cg-*Rag1^{tm1Mom} Prf1^{tm1Sdz}l*SzJ) or RG (BALB/cA-Rag2^{null}Il2rγ^{null} (BRG) or C57BL/6-Rag2^{null}Il2rγ^{null} (B6RG) mouse or SCID beige mouse. In one aspect the rat is a nude rat. In one preferred embodiment the non-human host is a BALB/cA-Rag2^{null}I12rγ^{null} (BRG) mouse (Traggiai E, at al, Science 304 (2004) 104 -107). Immunodeficient mice are described e.g. in Ryoji Ito et al, Cellular & Molecular Immunology 9 (2012) 208-214, and the respective references refferred cited therein. A variety of genetic mutations are known that impair immune functions in mice. Some of these mutant strains are widely used in research on cytokines to study a variety of physiologic and disease processes. Immunodeficient mouse models are useful for understanding mechanisms supporting allogeneic and xenogeneic tumor growth and progression. These mice are used as models of normal hematopoiesis, to study the ontogeny of immune system development, to investigate the growth of lymphomas and leukemic cell types, and to study the influences of cytokines and new therapeutic strategies. Acceptance of multiple tissue xenografts permits these mice to be used also as intermediate models for host-specific, fastidious organisms for which a small animal model has not been available previously. Genetic loci affecting immune responses include nu (nude), SCID (severe combined immunodeficiency), beige, and xid (X-linked immunodeficiency). The various mouse mutants have differing immunological properties and therefore permit complementary studies. The extent to which some of these mutations interfere with immune functions can vary with the genetic background. It has been shown, for example, that the immune defects in CH3 SCID mice are more severe than those in the original CB17 mice.

The term stem cell denotes, in a general manner, any cell which has not yet differentiated and which possesses the ability both to produce identical descendants and to differentiate into specific developmental lines. Adult stem cells have the function of maintaining cell number homeostasis in the tissue concerned, i.e. of replacing cells which have died. For this reason, stem cells are particularly to be found in tissues which are subjected to high stresses. Adult stem cells have been found in a very wide variety of tissues and organs, such as, for example, bone marrow, brain, liver, skin, intestine, cornea, etc. In the bone marrow, haematopoietic stem cells produce new cells continuously since these latter cells are constantly required in the blood owing to the limited life span of most of the cells. The starting point for the formation of blood cells is the pluripotent, undifferentiated haematopoietic stem cell which is still not determined for a specific function. When stem cells differentiate, precursor cells, which are unable to replicate themselves and only bring a specialized cell type to maturity, are formed first of all. Neither the pluripotent stem cell nor the different intermediate stages are able to fulfil cell-specific haematopoietic functions; it is only the cells which have matured which are able to do this. Progenitor cells which have entered upon a particular differentiation route then also keep to this route until maturation is achieved (commitment). The cell surface marker CD34, in particular, has been used in the past for isolating human hematopoietic stem cells which are expressing CD34, the so called human CD34 positive (CD34+) hematopoietic stem cells. In one embodiment the CD34 positive (CD34+) hematopoietic stem cells are human CD34 positive hematopoietic fetal liver stem cells.

The term "cancer immunotherapy" refers to the therapeutic agents or therapies which interact/ stimulate the immune system (e.g. specific immune effector cells) to reduce or prevent cancer, tumor growth and/ or metastasis. Cancer immunotherapy may be selected from the group of:
a) T cell engaging agents selected from agonistic antibodies which bind to human OX40, TO GITR, TO CD27, OR TO 4-1BB, und T-cell bispecific antibodies (e.g. T cell-engaging BiTE™ antibodies CD3-CD19, CD3-EpCam, CD3-EGFR), IL-2 (Proleukin), Interferon (IFN) alpha, antagonizing antibodies which bind to human CTLA-4 (e.g. ipilimumab), to PD-1, to PD-L1, to TIM-3, to BTLA, to VISTA, to LAG-3, or to CD25,
b) targeting immunosuppression: antibodies or small molecules targeting STAT3 or NFkB signaling, blocking IL-6, IL-17, IL-23,TNFa function,
c) cancer vaccines/enhance dendritic cell function: OncoVex (oncolytic virus secreting GM-CSF), an agonistic CD40 antibody, Toll-like receptor (TLR) ligands, TLR agonists, recombinant fusion protein encoding MAGE-A3, PROSTVAC; or
d) adoptive cell transfer: GVAX(prostate cancer cell line expressing GM-CSF), dendritic cell vaccine, adoptive T cell therapy, adoptive CAR T cell therapy.

The following examples and figures are provided to aid the understanding of the present invention.

### Example 1

### Reduction of mouse macrophages concomitantly with the generation of human macrophages in immunodeficient mice, reconstituted with human immune system.

### Generation and treatment of immunodeficient mice, reconstituted with human immune system (HIS mice)

### A) Generation of immunodeficient mice, reconstituted with human immune system (HIS mice)

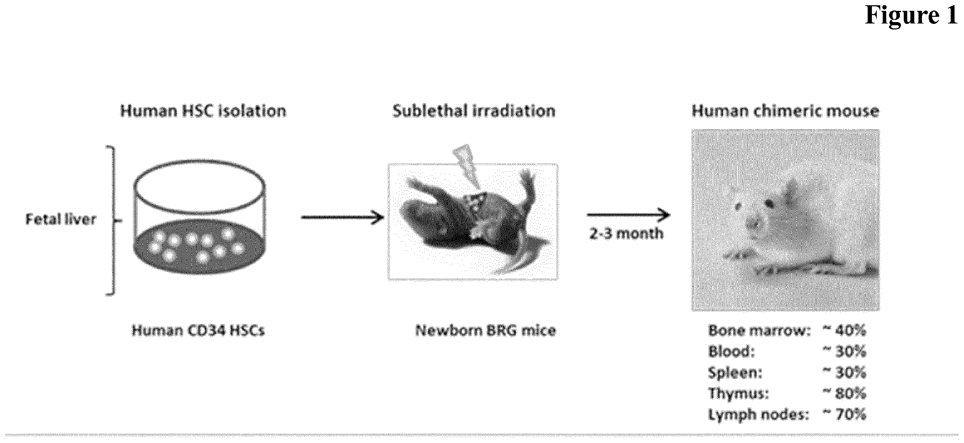

Pregnant female BRG mice as well as humanized BRG are kept under specific pathogen-free (SPF) conditions in the animal care facility at Roche Diagnostics in Penzberg. All mouse experiments are approved by the regional government of upper Bavaria (ref. 55.2-1-54-2532-156-11, ref. 55.2-1-54-2532.2-33-12). Timed-pregnant BRG mice are delivered at gestational day 17 and were monitored daily until birth. Newborn pups (within the first 3d of life) are sublethally irradiated with a dose of 2.5 Gy (250 rad) using a Cs-137 irradiator. 24h after irradiation ∼2x10⁵ human CD34 positive (CD34+) FL cells in 50 µl HSC medium are intrahepatically injected with a 29-gauge needle (Fig. 1). Overall humanization level and composition of human white blood cells is determined in peripheral blood of humanized mice 14-26 weeks after reconstitution. Mice with humanization level < 2% are termed "non-humanized" and excluded from the analysis.

### B) Anti-mouse-CSF-1R treatment of immunodeficient mice, reconstituted with human immune system (HIS mice)

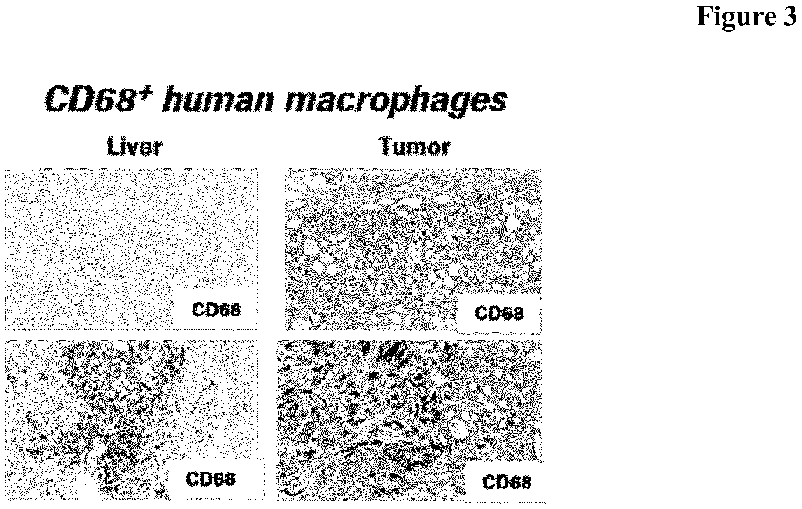

Depletion of mouse macrophages in humanized BRG mice can be performed at different time points after HSC transfer by four consecutive weekly intraperitoneal injections of a monoclonal anti-mouse CSF-1R antibody (2G2) at 30 mg/kg per injection (1q7dx4). Upon depletion of mouse monocytes and macrophages, murine M-CSF increases ∼ 1000 fold in sera of humanized BRG mice finally leading to increased levels of human blood monocytes and generation of tissue infiltrating human macrophages (Fig.2). Human tumor associated macrophages can be generated only if mouse macrophages have been depleted in humanized BRG mice (Fig.3). To do so, 2x10⁶ HT29 or 5x10⁶ Ovcar5 tumor cells (100 µl) are injected in the right flank of humanized BRG mice before weekly intraperitoneal injections of a monoclonal anti-mouse CSF-1R antibody (2G2) at 30 mg/kg per injection (1q7dx4). Tumor growth was monitored twice a week by caliper measurement and tumor volume was calculated using the following formula: volume = 0.5 × length2 × width. Moreover, body weight was measured daily using a laboratory scale. Generation of human M1 and M2 like tumor associated macrophages correlates with significantly reduced tumor growth and significantly increased frequencies of tumor infiltrating T cells. Inducible infiltration of human innate and adaptive immune effector cells in human Xenograft tumors provides a novel test system to evaluate new CIT drug candidates.

### C) Significant reduction of mouse macrophages in immunodeficient mice, reconstituted with human immune system/ Injection of an anti-mouse CSF1R antibody in HIS BRG mice

Injection of an anti-mouse CSF1R antibody in HIS BRG mice. Immunodeficient mice, reconstituted with human immune system mice were injected with 4 consecutive doses of 2G2 (anti-mouse CSF-1R) or mouse IgG1 isotype (MOPC21) at 30 mg/kg/week. Different parameters were analyzed before (t0) and during (t1-t4) the treatment in peripheral blood and at day of necropsy (t4) in spleen and bone marrow.

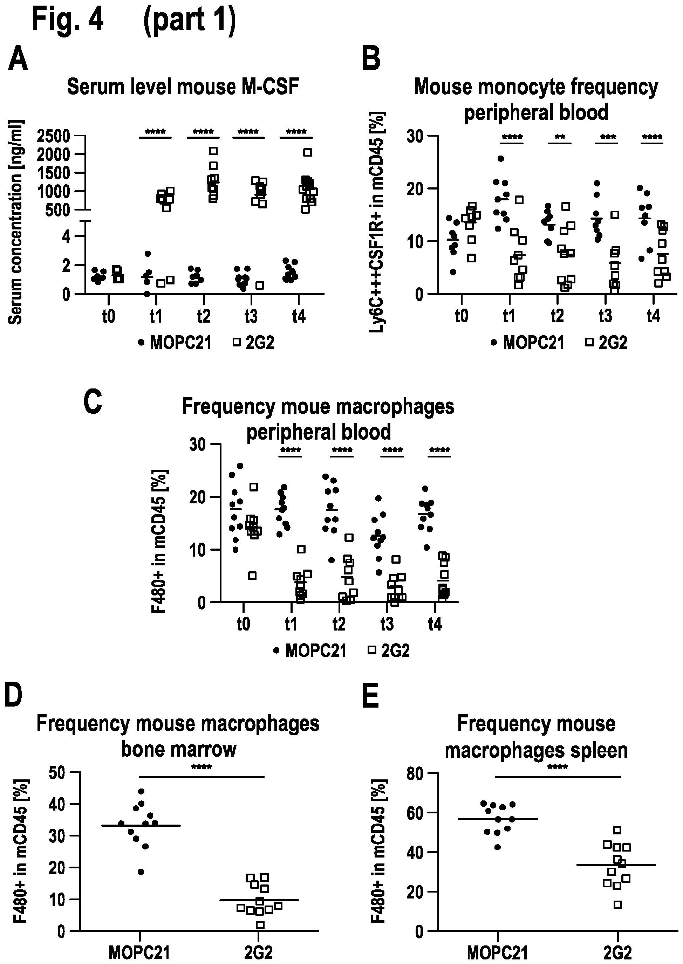

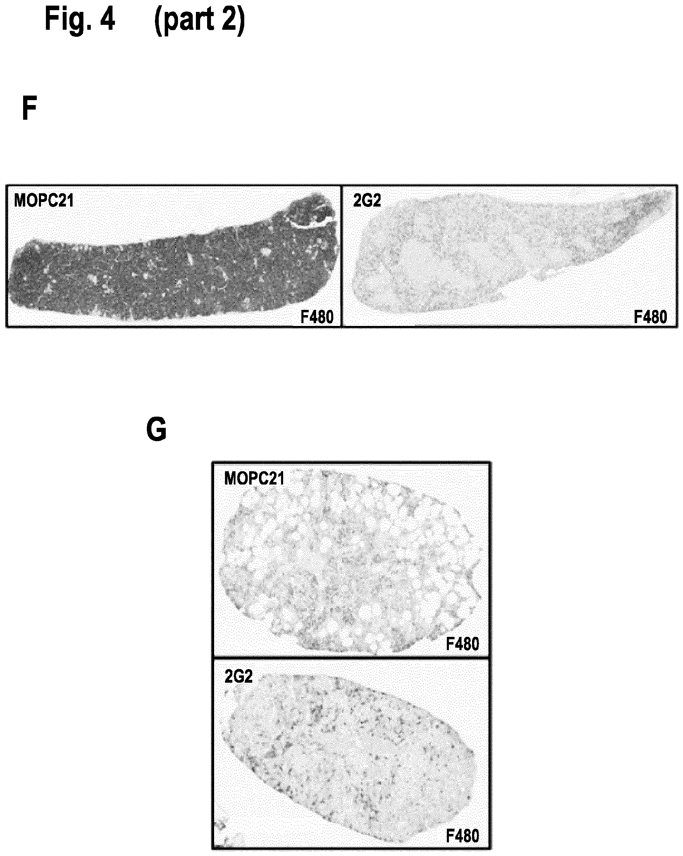

Results are shown in Figure 4
(A) Level of mouse M-CSF in serum of HIS BRG mice treated with 2G2 or MOPC21 (n=8-9 mice).
(B+C) Frequency of Ly6C⁺⁺⁺CSF1R⁺ mouse monocytes or macrophages within mouse CD45⁺ cells in peripheral blood of humanized mice injected with 4 doses of control (MOPC21) or anti-mouse CSF1R (2G2) antibody determined by FACS analysis (n= 8-10 mice; 2way Anova; ^{∗}P < 0.05).
(D+E) Frequency of Ly6C⁺⁺⁺CSF1R⁺ mouse monocytes or macrophages within mouse CD45⁺ cells in bone marrow or spleen of humanized mice injected with 4 doses of control (MOPC21) or anti-mouse CSF1R (2G2) antibody determined by FACS analysis (n= 8-9 mice; unpaired Student's t-test).
(F+G) Immunohistochemical staining of mouse F480 on representative spleen or bone marrow sections of humanized mice injected with 4 doses of control (MOPC21) or anti-mouse CSF1R (2G2) antibody.

**Table: Parameters to describe level of statistical significance**

| | | |
|---|---|---|
| < 0.0001 | Extremely significant | **** |
| 0.0001 to 0.001 | Extremely significant | *** |
| 0.001 to 0.01 | Very significant | ** |
| 0.01 to 0.05 | Significant | * |
| ≥ 0.05 | Not significant | ns |

## Claims

1. Use of an antibody which specifically binds to mouse colony-stimulating factor 1 receptor (CSF-1R) and which does not crossreact with human CSF-1R to stimulate the generation of human macrophages in immunodeficient mouse, wherein immunodeficient mouse is reconstituted with human CD34 positive hematopoietic stem cells and wherein the antibody binds to human CSF-1R with a binding affinity of KD-value of 1.0 x 10⁻⁶ mol/l or higher at 25°C.

2. The use according to claim 1, wherein in the mouse the mouse macrophages are reduced by at least 40% compared to an IgG control antibody.

3. The use according to any one of claims 1 to 2, wherein the human macrophages are generated in non-lymphoid tissues.

4. The use according to any one of claims 1 to 2, wherein the human macrophages are generated in lymphoid tissues.

5. The use according to any one of claims 1 to 4, wherein the mouse has been xenografted with human tumor cells.

6. The use according to claim 5, for the generation of human tumor associated macrophages (TAMs).

## Patentansprüche

1. Verwendung eines Antikörpers, der spezifisch an koloniestimulierenden-Faktor-1-Rezeptor (CSF-1R) der Maus bindet und der nicht mit menschlichem CSF-1R kreuzreagiert, zum Stimulieren der Erzeugung von menschlichen Makrophagen in einer immundefizienten Maus, wobei die immundefiziente Maus mit menschlichen CD34-positiven hämatopoetischen Stammzellen rekonstituiert wird und wobei der Antikörper mit einer Bindungsaffinität mit einem KD-Wert von 1,0 x 10⁻⁶ mol/l oder höher bei 25 °C an menschlichen CSF-1R bindet.

2. Verwendung nach Anspruch 1, wobei die Maus-Makrophagen in der Maus im Vergleich zu einem IgG-Kontrollantikörper um mindestens 40 % reduziert sind.

3. Verwendung nach einem der Ansprüche 1 bis 2, wobei die menschlichen Makrophagen in nicht lymphoiden Geweben erzeugt werden.

4. Verwendung nach einem der Ansprüche 1 bis 2, wobei die menschlichen Makrophagen in lymphoiden Geweben erzeugt werden.

5. Verwendung nach einem der Ansprüche 1 bis 4, wobei der Maus menschliche Tumorzellen xenotransplantiert wurden.

6. Verwendung nach Anspruch 5 zur Erzeugung menschlicher tumorassoziierter Makrophagen (TAMs).

## Revendications

1. Utilisation d'un anticorps qui se lie spécifiquement au récepteur du facteur de stimulation des colonies 1 (CSF-1R) de souris et qui ne réagit pas de manière croisée avec le CSF-1R humain pour stimuler la production de macrophages humains dans une souris immunodéficiente, dans laquelle la souris immunodéficiente est reconstituée avec des cellules souches hématopoïétiques CD34-positives humaines et dans laquelle l'anticorps se lie au CSF-1R humain avec une affinité de liaison de valeur KD de 1,0 x 10⁻⁶ mol/l ou plus à 25 °C.

2. Utilisation selon la revendication 1, dans laquelle dans la souris les macrophages de souris sont réduits d'au moins 40 % par comparaison à un anticorps IgG de contrôle.

3. Utilisation selon l'une quelconque des revendications 1 à 2, dans laquelle les macrophages humains sont produits dans des tissus non lymphoïdes.

4. Utilisation selon l'une quelconque des revendications 1 à 2, dans laquelle les macrophages humains sont produits dans des tissus lymphoïdes.

5. Utilisation selon l'une quelconque des revendications 1 à 4, dans laquelle la souris a reçu une xénogreffe de cellules tumorales humaines.

6. Utilisation selon la revendication 5, pour la production de macrophages associés aux tumeurs (TAM) humains.
